# EUROPEAN PATENT APPLICATION

(11) **EP 1 354 561 A1**
(43) Date of publication of application: **22.10.2003**
(21) Application number: 03009039.3
(22) Date of filing: 17.04.2003
(51) Int. Cl.: A61B 17/22, G10K 11/30, G10K 11/28, G10K 11/26, G10K 11/00, G10K 15/04, G10K 11/02

(54) **Apparatus for manipulating acoustic pulses**

(30) Priority: 17.04.2002 US 373362 P
(71) Applicant: Dornier MedTech Systems GmbH, 82234 Wessling (DE)
(72) Inventor: Eizenhöfer, Harald, 82229 Seefeld (DE)
(74) Representative: Grünecker, Kinkeldey, Stockmair & Schwanhäusser Anwaltssozietät

(57) **Abstract**

An apparatus and method for manipulating acoustic pulses is provided. The apparatus includes a wave-path with an acoustic network suspended within it, and the acoustic network includes at least one acoustic lens and at least one acoustic plate. The acoustic lens is structured to convert a plane pulse into a converging pulse, and the acoustic plate is structured to convert a single pulse into a split converging pulse. The method includes providing the foregoing wave-path and acoustic network, and converting a plane pulse to a split converging pulse by transmitting the plane pulse along the acoustic wave-path through the acoustic network.

## Description

### CROSS REFERENCE TO RELATED APPLICATION

This application claims priority to U.S. provisional application Serial No. 60/373,362, which was filed on April 17, 2002.

### FIELD OF THE INVENTION

The invention generally relates to the application of acoustic energy pulses to living tissue, and more particularly, to the manipulation of such acoustic pulses.

### BACKGROUND OF THE INVENTION

The application of acoustic energy pulses ("acoustic pulses") to living tissue, known as extracorporeal shockwave lithotripsy (ESWL), has become a popular approach to performing non-invasive surgical procedures on living beings. For example, acoustic pulses can be applied externally upon the living tissue of a person to facilitate the breakdown and removal of internal concretions, such as kidney stones. Another popular application of acoustic pulses to living tissue is known as extracorporeal shockwave therapy (ESWT), which is typically used to perform non-invasive internal treatment of orthopedic conditions such as plantar fasciitis (a painful, chronic disorder of soft tissue near the heel of the foot).

Typically, when acoustic pulses are applied to living tissue, so-called cavitation bubbles are generated in liquid portions of the tissue. These cavitation bubbles can contribute to the fragmentation of the targeted internal concretions, such as the kidney stones, by releasing energy in the vicinity of the concretions. Cavitation bubbles may have similar characteristics (such as appearance) to air bubbles that are produced by injecting air into a liquid, however, cavitation bubbles are produced as a result of the application of acoustic pulses. Also, similar to air bubbles, cavitation bubbles may grow or collapse in response to the modification or removal of the generating action (i.e., the application of acoustic pulses). For example, if the amount and/or intensity of acoustic pulses that are applied to living tissue is increased, the cavitation bubbles that are generated may increase in size and/or amount. Similarly, if the amount or intensity of acoustic pulses that are applied to living tissue is decreased, the cavitation bubbles that are generated may decrease in size and/or amount, or the bubble generation may fall below a productive threshold (e.g., the bubble generation may cease).

Manipulation of cavitation bubbles is an important aspect to the effective performance of non-invasive internal surgery or therapy by the external application of acoustic pulses to living tissue For example, the increase or decrease in the size and/or amount of cavitation bubbles that are generated, or the collapse of cavitation bubbles that have been generated, may be needed for effective surgical or therapeutic performance. Typically, such manipulation of cavitation bubbles can be accomplished by manipulating the acoustic pulses that generate the cavitation bubbles, and there are several existing approaches to manipulating acoustic pulses for this purpose. One existing approach involves the generation of two or more separate, complete acoustic pulses to cause the manipulation of cavitation bubbles. This approach is typically performed with two separate sources of acoustic pulses or with a source that produces two complete acoustic pulses that are separated by a time delay period. However, this approach has shortcomings, due to its requirement to produce two or more separate, complete acoustic pulses to manipulate cavitation bubbles. For example, the time delay that can be established between acoustic pulses that are generated by this approach is typically constrained and/or difficult to consistently maintain. Furthermore, a device that is configured to implement this approach typically has a high complexity and cost and a large physical size, which thereby limits its clinical applications.

Another existing approach to manipulating acoustic pulses for the purpose of manipulating cavitations bubbles involves reflecting acoustic pulses upon a series of reflectors in order to separate the pulses into sections that are staggered by various time delays. The time delays between the staggered pulse sections are typically dependent on the position and/or orientation of the reflectors. However, this approach also has shortcomings. For example, this approach is typically limited to devices that use a point shock wave source to produce acoustic pulses. Furthermore, this approach also typically causes an increase in the complexity, cost, and physical size of the device used to implement it.

Still another existing approach involves a shockwave source, such as a spark gap, a focusing reflector, such as a hollow ellipsoid that is filled with a propagation medium, and a layer of material that is positioned so that shockwaves produced by the source are reflected to it by the focusing reflector. The layer of material in this approach typically has an acoustic impedance that differs from that of the propagation medium. However, this approach is typically limited to providing pulse trains of shockwaves.

Based on the above discussion, it should be appreciated that there is a need in the art for an invention that can manipulate acoustic pulses to manipulate cavitation bubbles without the need to produce two or more separate, complete acoustic pulses. Furthermore, there is a need in the art for an invention that can manipulate acoustic pulses without being limited to the use of point shock wave sources or reflector networks. Finally, there is a need in the art for an invention that can manipulate acoustic pulses without the need to use devices that are large in physical size and high in complexity and cost, which limits their clinical applications.

### SUMMARY OF THE INVENTION

The present invention is generally directed to an apparatus and method for manipulating acoustic pulses. In one aspect, the invention provides an apparatus that includes an acoustic wave-path. The acoustic wave-path has an acoustic impedance and is structured to allow acoustic pulses to travel along it. An acoustic network is suspended within the acoustic wave-path. The acoustic network includes at least one acoustic lens that also has an acoustic impedance. The acoustic lens is structured to convert an acoustic plane pulse into an acoustic converging pulse, when at least part of the acoustic plane pulse passes through the acoustic lens. The acoustic network also includes at least one acoustic plate. The acoustic plate also has an acoustic impedance. Furthermore, the acoustic plate is structured to convert an acoustic converging pulse into a split acoustic converging pulse, when at least part of the acoustic converging pulse passes through the acoustic plate.

In another aspect of the present invention, a method for manipulating an acoustic pulse is provided. Broadly described, the method includes: providing an acoustic wave-path that has an acoustic impedance and is structured to allow the travel of acoustic pulses; providing at least one acoustic lens that is suspended within the acoustic wave-path and has an acoustic impedance, and that is structured to convert an acoustic plane pulse into an acoustic converging pulse, when at least part of the acoustic plane pulse travels through the acoustic lens; providing at least one acoustic plate that is suspended within the acoustic wave-path and has an acoustic impedance, and that is structured to convert an acoustic converging pulse into a split acoustic converging pulse that has at least two components, when at least part of the acoustic converging pulse travels through the acoustic plate; transmitting an acoustic plane pulse along the acoustic wave-path; converting the acoustic plane pulse into an acoustic converging pulse by transmitting at least part of the acoustic plane pulse through the acoustic lens; and, converting the acoustic converging pulse into a split acoustic converging pulse by transmitting at least part of the acoustic converging pulse through the acoustic plate.

These and other aspects of the invention will be described further in the detailed description below in connection with the drawings and the claims.

### BRIEF DESCRIPTION OF THE DRAWINGS

Fig. 1 is a block diagram illustrating an exemplary application of an acoustic pulse to a living being in accordance with the present invention.
Fig. 2 is a block diagram illustrating an exemplary architecture of the acoustic energy source introduced in Fig. 1.
Fig. 3A is an exemplary cross-sectional view of an exemplary general architecture of the present invention for manipulating acoustic pulses.
Fig. 3B is an exemplary bottom view of an exemplary general architecture of the present invention for manipulating acoustic pulses.
Fig. 4A is an exemplary cross-sectional view of a first exemplary embodiment of the present invention for manipulating acoustic pulses.
Fig. 4B is an exemplary bottom view of a first exemplary embodiment of the present invention for manipulating acoustic pulses.
Fig. 5A is an exemplary cross-sectional view of a second exemplary embodiment of the present invention for manipulating acoustic pulses.
Fig. 5B is an exemplary bottom view of a second exemplary embodiment of the present invention for manipulating acoustic pulses.
Fig. 6A is an exemplary cross-sectional view of a third exemplary embodiment of the present invention for manipulating acoustic pulses.
Fig. 6B is an exemplary bottom view of a third exemplary embodiment of the present invention for manipulating acoustic pulses.
Fig. 7A is an exemplary cross-sectional view of a fourth exemplary embodiment of the present invention for manipulating acoustic pulses.
Fig. 7B is an exemplary bottom view of a fourth exemplary embodiment of the present invention for manipulating acoustic pulses.

### DETAILED DESCRIPTION OF THE INVENTION

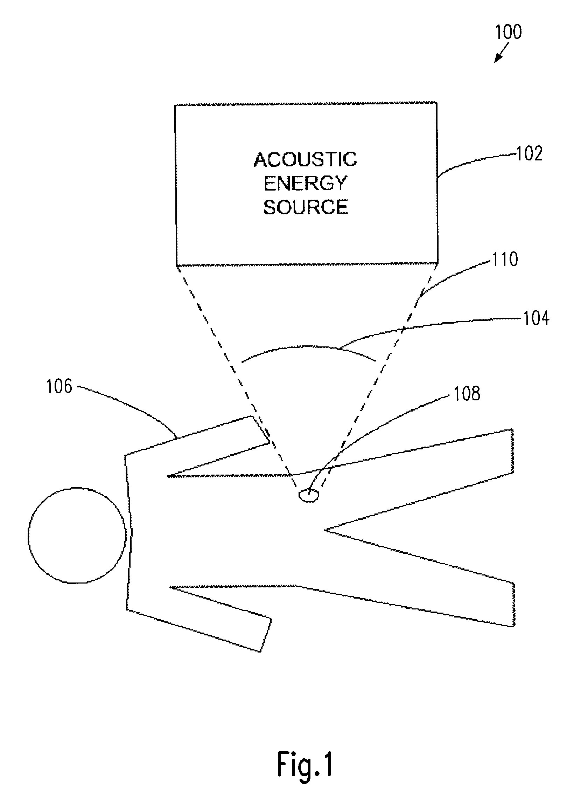

Referring now to the drawings, in which like numerals represent like elements throughout the several figures, aspects of the present invention will be described. Fig. 1 is a block diagram 100 illustrating an exemplary application of an acoustic pulse to a living being in accordance with the present invention. The exemplary block diagram 100 includes an acoustic energy source 102. The acoustic energy source is capable of generating an acoustic pulse 104, which is typically transmitted or propagated along a wave-path (or beam-path) 110. The acoustic pulse 104 is typically directed to a target 108 on or within a living being 106, such as a human person.

An important aspect of the application of an acoustic pulse 104 to a target 108 within a living being 106 is that the surgical manipulation of the target 108 can be performed non-invasively, that is, without the need to physically penetrate the living being 106 with surgical instruments or devices. For example, the application of one or more ultrasonic acoustic pulses 104 to a target 108 within a living being 106 can produce cavitation bubbles (not depicted) that in turn release localized mechanical energy upon the target 108. The target 108 may be a concretion in a human person, such as one or more kidney stones, and one or more acoustic pulses 104 may be applied to this target concretion 108. Typically, the result of applying such acoustic pulses 104 and creating such cavitation bubbles, as described above, is to cause the fracture and/or erosion of the target concretion 108 for the purpose of removing it from the living being 106.

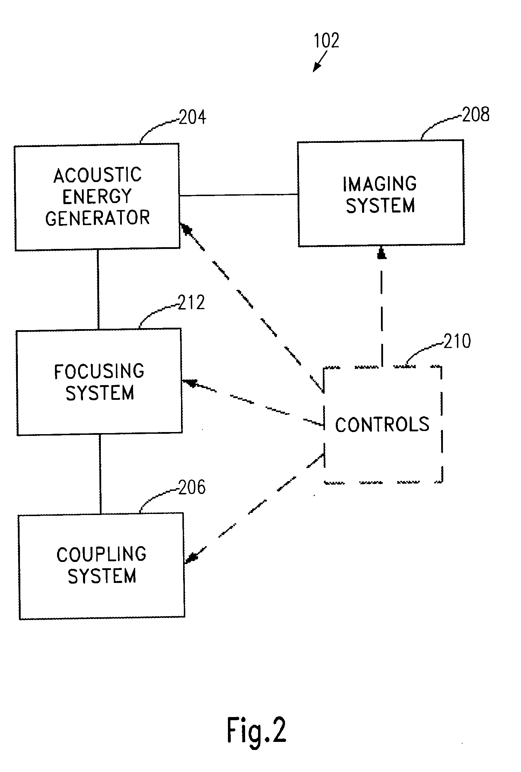

The acoustic energy source 102 typically includes several components, which will be described below with respect to Fig. 2. The acoustic energy source 102 may be provided, for example, by a shock wave lithotripter system. In this regard, components of the acoustic energy source 102 may include a wave-path 110, which is depicted in Fig. 1 separately for illustrative purposes. As will be discussed with respect to Fig. 2 and subsequent figures, the wave-path 110 may include a medium, such as a liquid, and the wave-path 110 may include the structure of a waveguide that is capable of directing an acoustic pulse 104.

The acoustic pulse 104 may have various forms or configurations and may be manipulated from one such form to another. For example, the acoustic pulse 104 may have the form of a plane pulse (i.e., planar in shape; not depicted). As another example, the acoustic pulse 104 may have the form of a converging pulse, as depicted in Fig. 1. Furthermore, the acoustic pulse 104 may be manipulated, for example, from the form of a plane pulse to that of a converging pulse. As will be discussed below with respect to various embodiments of the present invention, the manipulation of the acoustic pulse 104 can be made by the use of various components suspended within the wave-path 110 or some other appropriate component of the acoustic energy source 102.

Fig. 2 is a block diagram illustrating an exemplary architecture of the acoustic energy source 102 introduced in Fig. 1. In this regard, the acoustic energy source 102 can include an acoustic energy generator 204. The acoustic energy generator 204 can be provided by a shock wave generator (not depicted) that operates on the principle of electro-hydraulic, piezoelectric, or electromagnetic energy generation. Typically, the acoustic energy generator 204 operates in an ultrasonic frequency range (i.e., > 18 kHz). The acoustic energy source 102 can also include a focusing system 210. This focusing system 210 may include a wave-path 110, as discussed above. Typically, the focusing system 210 operates to direct an acoustic pulse 104, or other acoustic energy form, from the acoustic energy generator 204 to a target 108.

The acoustic energy source 102 may also include a coupling system 206, which couples the acoustic pulse 104 or other energy transmitted from the acoustic energy generator 204 to the target 108. In this regard, the coupling system 206 may be a part of the focusing system 210 in some embodiments of the invention. For example, the coupling system 206 may be the liquid medium portion (not depicted) of the wave-path 110 described above.

The acoustic energy source 102 may also include an imaging system 208. The imaging system 208 may be used to obtain a local image or representation of the target 108 and surrounding tissue within a living being 106. This image or representation can be used to facilitate the appropriate application of the acoustic energy 104 to the target 108. The imaging system 208 may be provided, for example, by a system for sonography or fluoroscopy.

One or more controls 212 may also be included in the acoustic energy source 102. The controls 212 can be connected, for example electrically and/or mechanically, to one or more of the other components of the acoustic energy source 102. Thus, the controls may be connected to the acoustic energy generator 204, the focusing system 210, the coupling system 206, and/or the imaging system 208. The controls 212 can transmit and/or receive signals from these other components of the acoustic energy source 102 in order to facilitate the operation of the acoustic energy source 102. For example, the controls 212 may transmit one or more electrical and/or mechanical signals to the acoustic energy generator 204 to cause it to produce one or more acoustic pulses 104 in a particular form or configuration. The controls 212 may be provided by various forms of electrical and /or mechanical devices (not depicted), such as a computing device or an electro-mechanical actuation device.

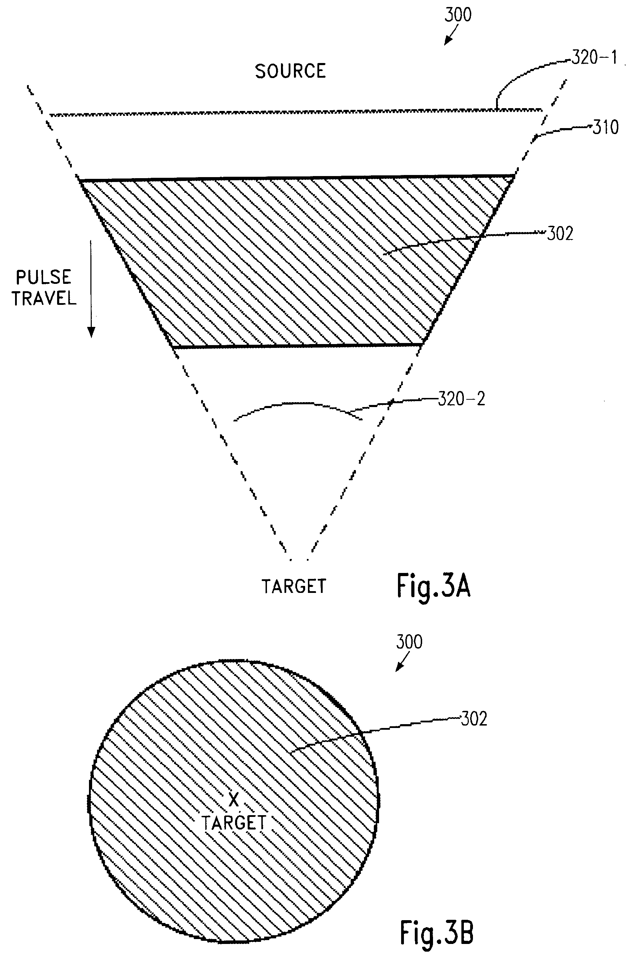

Fig. 3A and Fig. 3b are an exemplary cross-sectional view and exemplary bottom view, respectively, of an exemplary general architecture 300 of the present invention. In this regard, the exemplary architecture 300 includes a wave-path 310, which may be similar to the wave-path 110 or a component of the focusing system 210, which were described above for Figs. 1 and 2, respectively. Thus, the wave-path 310 may include a medium, such as a liquid, gas, or solid material. Furthermore, the wave-path 310 may include a waveguide structure that is capable of directing an acoustic pulse 320 along it. Typically, the wave-path 310 possesses some structure or form that allows the travel or propagation of one or more acoustic pulses along it, for example, within a medium.

The wave-path 310 typically has the property of an acoustic impedance, which can be described as a product of the sound velocity characteristic of the wave-path 310 and the density of the wave-path 310. Thus, an acoustic impedance typically includes a sound velocity component and a density component. The acoustic impedance of the wave-path 310 can vary based on a variance in the sound velocity component and/or the density. For example, depending on the sound velocity and density components of the material that the wave-path 310 includes, such as a liquid, the wave-path 310 can possess a particular acoustic impedance.

The general architecture 300 of the present invention also includes an acoustic network 302, which is typically suspended within the wave-path 310. The acoustic network 302 may be suspended within the wave-path 310 in any manner, for example by mechanical connections to a surface that may be part of the wave-path 310 or other components of an acoustic pulse generator 102. In accordance with exemplary embodiments of the present invention, the acoustic network 302 typically includes one or more components that can be used to manipulate the shape and/or travel of an acoustic pulse 320 along the wave-path 310. For example, the acoustic network 302 may include one or more acoustic lenses and/or one or more acoustic plates (not shown) which are structured to manipulate acoustic pulses 320 that pass through them.

The one or more acoustic lenses and acoustic plates that are included in the acoustic network 302 may be structured in various shapes and formed of various materials. For example, the one or more acoustic lenses and acoustic plates may be structured to include one or more convex and/or concave surfaces. Moreover, the one or more acoustic lenses and acoustic plates may be constructed of materials such as, but not limited to, various polymers, silicon, various synthetic rubbers, polystyrene, polyurethane, or a liquid. Other materials that are known in the art may also be used to construct the one or more acoustic lenses and acoustic plates of the acoustic network 302 as well. The one or more acoustic lenses and acoustic plates of the acoustic network 302 can possess various acoustic impedances, which can vary based on the sound velocity and density characteristics of these components, as discussed above.

As depicted in Figs. 3A and 3B, the wave-path 310 is typically oriented between a source of acoustic pulses 320, such as an acoustic energy generator 204, and a target 108 that the acoustic pulses are to be applied to, such as a concretion within a living being 106. Furthermore, the wave-path 310 may be shaped to focus the acoustic pulse 320 upon the target 108. In this regard, an acoustic pulse 320 can be transmitted along the wave-path 310 through the acoustic network 302 to a target 108. For example, an acoustic plane pulse 320-1 can be transmitted along the wave-path 310 from a source. The acoustic plane pulse 320-1 passes through the acoustic network 302 as it travels along the wave-path 310 and arrives at a target in the form of an acoustic converging wave 320-2.

Depending on the components that are included in the acoustic network 302 and their acoustic impedance properties, an acoustic pulse that travels along the wave-path 310 and through the acoustic network 302 can be manipulated to change its form, shape, and/or rate of travel. For example, as depicted in Fig. 3A, an acoustic plane pulse 320-1 that is transmitted along the wave-path 310 can be manipulated into the form of an acoustic converging pulse 320-2 as it passes through the acoustic network 302. Furthermore, although not depicted in Fig. 3A, the acoustic plane pulse 320-1 may be divided into two or more components as it passes through the acoustic network 302. Moreover, the rate of travel or propagation of these two or more components of the acoustic plane pulse 320-1 may be delayed and/or advanced with respect to one another as they pass through the acoustic network 302. Such manipulations of acoustic pulses 320 may also depend on the acoustic impedance properties of the acoustic network 302 in comparison to the acoustic impedance properties of the wave-path 310. More specific examples of such manipulations of acoustic pulses and exemplary embodiments of the invention that can perform such manipulations will be discussed below with respect to subsequent figures.

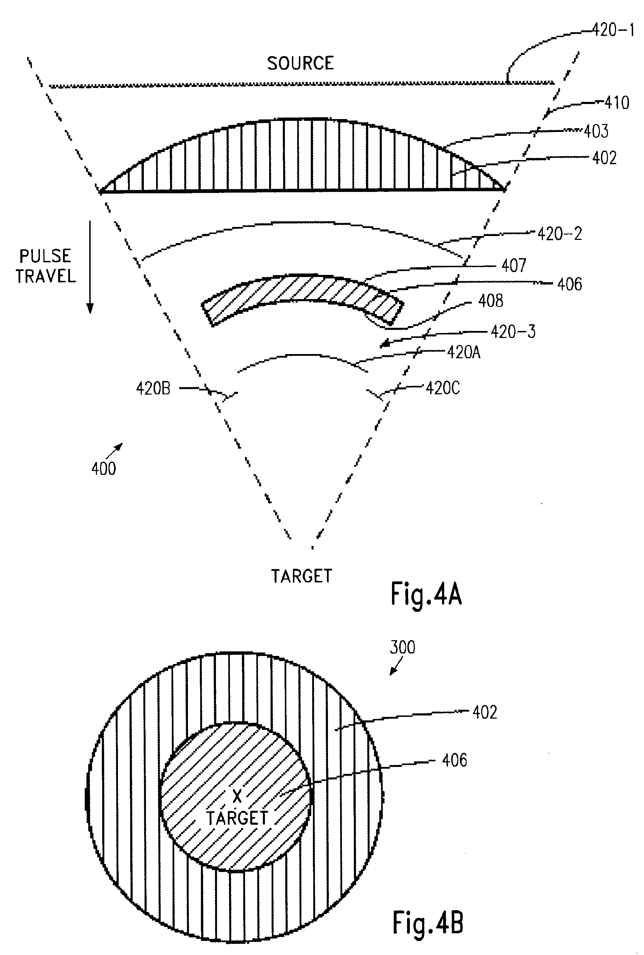

Fig. 4A and Fig. 4b are an exemplary cross-sectional view and exemplary bottom view, respectively, of a first exemplary embodiment 400 of the present invention. Similar to the general architecture 300 described above, the exemplary embodiment 400 includes a wave-path 410. The wave-path 410 is at least substantially similar to the wave-path 310 described above with respect to Fig. 3A. For example, the wave-path 410 may include a medium, such as a liquid, gas, or solid, and typically possesses some structure or form that allows the travel or propagation of one or more acoustic pulses 420 along it, for example, within the medium. Moreover, the wave-path 410 may include a waveguide structure that is capable of directing an acoustic pulse 420, and typically possesses an acoustic impedance that includes a sound velocity component and a density component.

Several components 402, 406 are suspended within the wave-path 410 and form an acoustic network, similar to the acoustic network 302 described above, that is capable of converting an acoustic plane pulse 420-1 into an acoustic converging pulse 420-3 with two or more acoustic converging pulse components 420A, 420B, 420C. Moreover, these acoustic network components 402, 406 within the wave-path 410 are capable of delaying the travel of one acoustic converging pulse component 420A with respect to the travel of the other components 420B, 420C along the wave-path 410. Similar to the one or more acoustic lenses and acoustic plates described with respect to the acoustic network 302 of Figs. 3A-3B, these acoustic network components 402, 406 typically possess an acoustic impedance property, which includes a sound velocity component and a density component. Furthermore, these acoustic network components 402, 406 can be constructed of materials such as, but not limited to, various polymers, silicon, various synthetic rubbers, polystyrene, polyurethane, or a liquid.

One component suspended in the wave-path 410 includes an acoustic lens 402. The acoustic lens 402 includes at least one convex surface 403 and, therefore, might be described as a convex or plano-convex acoustic lens. In the first exemplary embodiment 400, the sound velocity component of the acoustic lens 402 is typically slower than the sound velocity component of the wave-path 410. As a result, an acoustic pulse typically travels through the acoustic lens 402 at a slower rate of travel than it travels through the wave-path 410 in general. Due, at least in part, to the convex surface 403 of the acoustic lens 402 and its slower sound velocity component, the acoustic lens 402 is structured to convert an acoustic plane pulse 420-1 that is transmitted through it into an acoustic converging pulse 420-2, as exemplarily illustrated in Fig. 4A.

Another component suspended in the wave-path 410 includes an acoustic plate 406. The acoustic plate 406 is typically positioned adjacent to the acoustic lens 402. In regard to the intended meaning of the term "adjacent," with respect to the present invention, the acoustic plate 406 may be positioned directly next to the acoustic lens 402 or there may be other components positioned between the acoustic plate 402 and the acoustic lens 406. Furthermore, the distance between the position of the acoustic plate 406 and the acoustic lens 402 may vary.

The acoustic plate 406 can include a convex surface 407 and a concave surface 408. As depicted in Fig. 4A, the acoustic plate 406 may extend only partially along a cross-sectional area of the wave-path 410. As a result, only a portion of an acoustic pulse, such as the acoustic converging pulse 420-2, may pass through the acoustic plate 406, while other portions of the acoustic pulse pass only through the wave-path 410. In the exemplary embodiment 400, the acoustic plate 406 typically also possesses a sound velocity component that is slower than the sound velocity component of the wave-path 410. In this regard, the acoustic plate 406 might be referred to as an acoustic retarding plate.

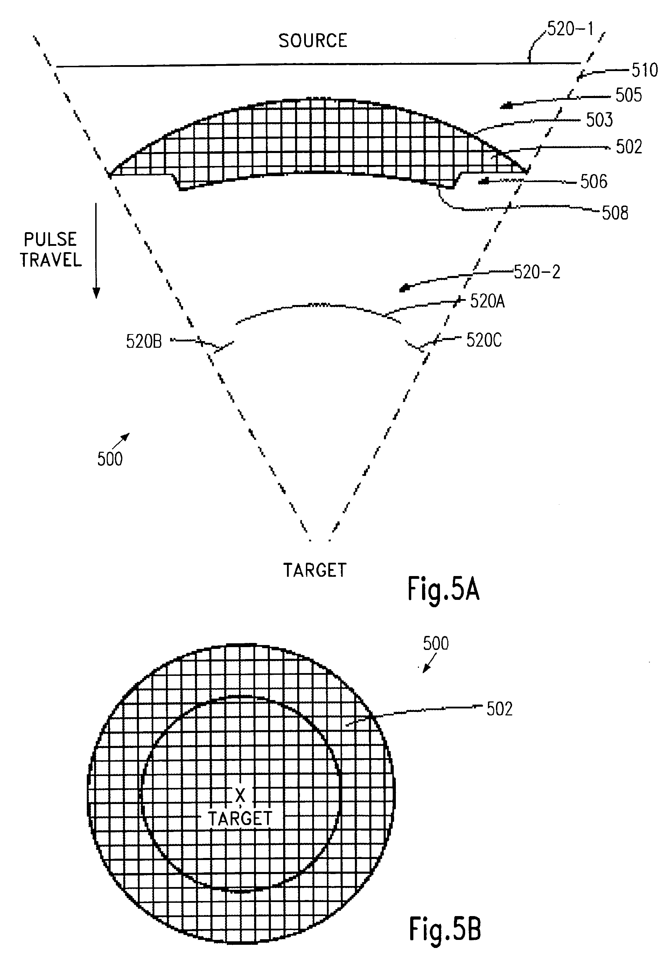

As a result, at least in part, of the convex and concave surfaces 407, 408 of the acoustic plate 406, its position in the wave-path 410, and its slower sound velocity component, the acoustic plate 406 is structured to convert an acoustic converging pulse 420-2 that passes through it into a split acoustic converging pulse 420-3 that has two or more components 420A, 420B, 420C, as exemplarily illustrated in Fig. 4A. Moreover, as depicted, the travel of the acoustic converging pulse component 420A that passes through the acoustic plate 406 is delayed with respect to the travel of the other pulse components 420B, 420C that do not pass through the acoustic plate 406. As will be discussed below with respect to Figs. 5A-5B, the acoustic network components 402, 406 can be integrated together or formed in a single piece of material in some embodiments of the present invention.

Fig. 5A and Fig. 5B are an exemplary cross-sectional view and exemplary bottom view, respectively, of a second exemplary embodiment 500 of the present invention. This second exemplary embodiment 500 includes a wave-path 510 that is at least substantially similar to the wave-path 410 described above. Furthermore, the wave-path 510 includes a component 502 suspended within it that forms an acoustic network that is capable of converting an acoustic plane pulse 520-1 into a split acoustic converging pulse 520-2 with two or more components 520A, 520B, 520C. Also similar to the wave-path 410 and the acoustic network components 402, 406 suspended within it, the acoustic network component 502 suspended within the wave-path 510 is capable of delaying the travel of one acoustic converging pulse component 520A with respect to the travel of the other components 520B, 520C along the wave-path 510.

In contrast to the acoustic network components 402, 406, the acoustic network component 502 performs the conversion of an acoustic plane pulse 520-1 to a split acoustic converging pulse 520-2 without the pulse 520-1 passing through two separately positioned components. In this regard, the acoustic network component 502 includes an acoustic lens portion 505 and an acoustic plate portion 506 that are either integrated together or formed in a single piece of material. As depicted, the acoustic lens portion 505 typically includes at least one convex surface 503, and the acoustic plate portion 506 includes at least one non-uniformly stepped surface 508. In some embodiments, this non-uniformly stepped, e.g. unsteady stepped surface 508 may be at least substantially concave shaped, as depicted for example in Fig. 5A. The term unsteady stepped may be understood in a mathematical sense, e.g. the surface may have one or more erratic or discontinuous passings.

When the acoustic lens portion 505 and the acoustic plate portion 506 are integrated together, the acoustic network component 502 is at least substantially similar in structure to positioning the acoustic lens 402 and the acoustic plate 406 of Fig. 4A directly next to each other. For example, the structure of the acoustic network component 502 is at least substantially similar to the structure formed by affixing the acoustic lens 402 and the acoustic plate 406 together mechanically (for example) to form an integrated structure from the two individual components. Thus, when integrated together, the acoustic lens portion 505 and the acoustic plate portion 506 can be formed of different materials, such as those discussed above, inhomogeneously distributed within the wave-path 510. When integrated together, the acoustic lens portion 505 and the acoustic plate portion 506 combine at least substantially similar acoustic pulse manipulation capabilities to those of the separately positioned acoustic lens 402 and acoustic plate 406 described above for Fig. 4A. Thus, when integrated together, the acoustic lens portion 505 and the acoustic plate portion 506 each typically possess a sound velocity component that is slower than the sound velocity component of the wave-path 510.

When the acoustic lens portion 505 and the acoustic plate portion 506 are formed in a single piece of material, the resulting acoustic network component 502 is also at least substantially similar in structure to positioning the acoustic lens 402 and the acoustic plate 406 directly next to each other. However, the acoustic network component 502 is formed of one type of material in this case, such as those discussed above. Therefore, the acoustic lens portion 505 and the acoustic plate portion 506 typically possess the same sound velocity property, which is typically slower than that of the wave-path 510 in this embodiment of the present invention. Moreover, when the acoustic lens portion 505 and the acoustic plate portion 506 are formed in a single piece of material, the resulting acoustic network component 502 also combines at least substantially similar acoustic pulse manipulation capabilities to those of the separately positioned acoustic lens 402 and acoustic plate 406 described above.

Forming the acoustic network component 502 by either integrating together the acoustic lens portion 505 and the acoustic plate portion 506 or forming the portions 505, 506 in the same piece of material can offer benefits, such as in manufacturing and implementing the exemplary embodiment 500. For example, the single material formation of the acoustic network component 502 can provide a simple and less expensive manufacturing process in comparison to manufacturing separate components 402, 406. As another example, the integrated formation of the acoustic network component 502 might be implemented in smaller physical structures than the separate components 402, 406. However, implementing the separate components 402, 406 can offer superceding benefits in certain applications and/or under certain considerations and requirements.

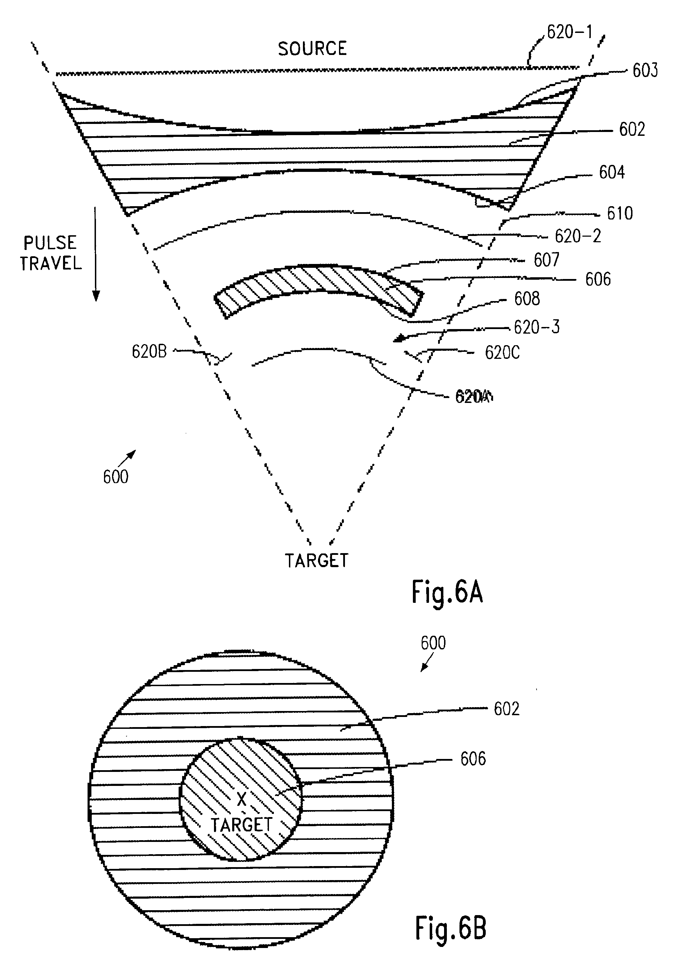

Fig. 6A and Fig. 6b are an exemplary cross-sectional view and exemplary bottom view, respectively, of a third exemplary embodiment 600 of the present invention. The exemplary embodiment 600 includes a wave-path 610 that is at least substantially similar to the wave-path 310 described above with respect to Fig. 3A. In this regard, the wave-path 610 may include a medium, such as a liquid, gas, or solid, and the waveguide 610 typically possesses a structure or form that allows the travel of one or more acoustic pulses 620 along it, for example, within the medium. Furthermore, the wave-path 610 may include a waveguide structure that is capable of directing an acoustic pulse 620, and the wave-path 610 typically possesses an acoustic impedance that includes a sound velocity component and a density component.

Similar to the exemplary embodiment 400 described above with respect to Figs. 4A-4B, the exemplary embodiment 600 includes several components 602, 606 suspended within the wave-path 610 that form an acoustic network. This acoustic network is similar to the acoustic network 302 described above for Fig. 3A and is capable of converting an acoustic plane pulse 620-1 into a split acoustic converging pulse 620-3 with two or more acoustic converging pulse components 620A, 620B, 620C. However, in contrast to the components 402, 406 of Figs 4A-4B, the acoustic network components 602, 606 are capable of advancing the travel of one acoustic converging pulse component 620A with respect to the travel of the other components 620B, 620C along the wave-path 610. The acoustic network components 602, 606 typically possess an acoustic impedance property, which includes a sound velocity component and a density component, similar to the properties described above with regard to the acoustic network 302 of Figs. 3A-3B. Moreover, the acoustic network components 602, 606 can be constructed of materials such as, but not limited to, various polymers, silicon, various synthetic rubbers, polystyrene, polyurethane, or a liquid.

One of the acoustic network components suspended within the wave-path 610 includes an acoustic lens 602. The acoustic lens 602 typically includes at least a first concave surface 603 and, in this exemplary embodiment 600, also includes a second concave surface 604, as depicted. Therefore, the acoustic lens 602 might be referred to as a concave or plano-concave acoustic lens. In the exemplary embodiment 600, the sound velocity component of the acoustic lens 602 is typically faster than the sound velocity component of the wave-path 610. Therefore, an acoustic pulse typically travels through the acoustic lens 602 at a faster rate of travel than it travels through the wave-path 610 in general. As a result, at least in part, of the concave surfaces 603, 604 of the acoustic lens 602 and its faster sound velocity component, the acoustic lens 602 is structured to convert an acoustic plane pulse 620-1 that is transmitted through it into an acoustic converging pulse 620-2, as exemplarily illustrated in Fig. 6A.

Another acoustic network component that is suspended in the wave-path 610 includes an acoustic plate 606. Typically, the acoustic plate 606 is positioned adjacent to the acoustic lens 602. In regard to the intended meaning of the term "adjacent," with respect to the present invention, the acoustic plate 606 may be positioned directly next to the acoustic lens 602 or there may be other components positioned between the acoustic plate 602 and the acoustic lens 606. Also, the distance between the position of the acoustic plate 606 and the acoustic lens 602 may vary.

Similar to the acoustic plate 406 of Fig. 4A, the acoustic plate 606 can include a convex surface 607 and a concave surface 608. Furthermore, the acoustic plate 606 may extend only partially along a cross-sectional area of the wave-path 610, as depicted in Fig. 6A. As a result of this positioning of the acoustic plate 606, only a portion of an acoustic pulse, such as the acoustic converging pulse 620-2, may pass through the acoustic plate 606, while other portions of the acoustic pulse pass only through the wave-path 610. The acoustic plate 606 typically possesses a sound velocity component that is faster than the sound velocity component of the wave-path 610, in the exemplary embodiment 600. Therefore, the acoustic plate 606 might be referred to as an acoustic accelerating plate.

Due, at least in part, to the convex and concave surfaces 607, 608 of the acoustic plate 606 and its faster sound velocity component, the acoustic plate 606 is structured to convert an acoustic converging pulse 620-2 that passes through it into a split acoustic converging pulse 620-3 that has two or more components 620A, 620B, 620C, as exemplarily illustrated in Fig. 6A. Additionally, the travel of the acoustic converging pulse component 620A that passes through the acoustic plate 406 is advanced with respect to the travel of the other components 620B, 620C that do not pass through the acoustic plate 606, as depicted in Fig. 6A. As will be discussed below with respect to Figs. 7A-7B, the acoustic network components 602, 606 can also be integrated together or formed in a single piece of material in some embodiments of the present invention.

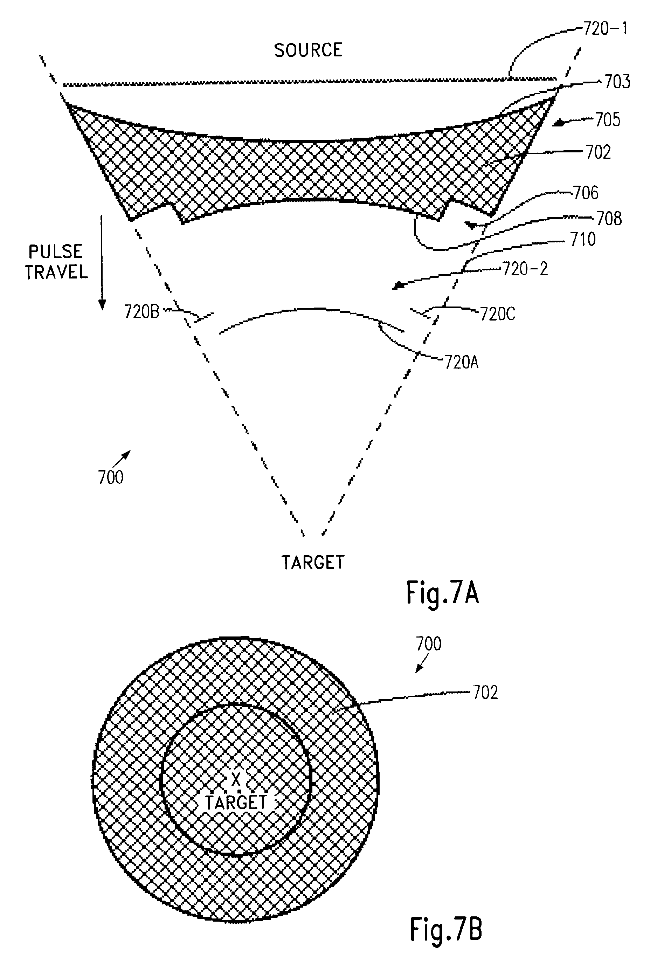

Fig. 7A and Fig. 7B are an exemplary cross-sectional view and exemplary bottom view, respectively, of a fourth exemplary embodiment 700 of the present invention. This exemplary embodiment 700 also includes a wave-path 710, which is at least substantially similar to the wave-path 610 described above. The wave-path 710 includes a component 702 suspended within it that forms an acoustic network that is capable of converting an acoustic plane pulse 720-1 into a split acoustic converging pulse 720-2 with two or more components 720A, 720B, 720C. Similar to the acoustic network components 602, 606 suspended within the wave-path 610, the acoustic network component 702 is capable of advancing the travel along the wave-path 710 of one acoustic converging pulse component 720A with respect to the travel of the other pulse components 720B, 720C.

In contrast to the acoustic network components 602, 606, and similar to the acoustic network component 502, the acoustic network component 702 performs the conversion of an acoustic plane pulse 720-1 to a split acoustic converging pulse 720-2 without passing through two separately positioned components. The acoustic network component 702 includes an acoustic lens portion 705 and an acoustic plate portion 706, in this regard, that are either integrated together or formed in a single piece of material. The acoustic lens portion 705 typically includes at least one concave surface 703, and the acoustic plate portion 706 includes at least one non-uniformly stepped surface 708. In some embodiments, this non-uniformly stepped, e.g. unsteady stepped surface 708 may be at least substantially concave shaped, as depicted for example in Fig. 7A. The term unsteady stepped may be understood in a mathematical sense, e.g. the surface may have one or more erratic or discontinuous passings.

Similar to the acoustic network component 502, when the acoustic lens portion 705 and the acoustic plate portion 706 are integrated together, the acoustic network component 702 is at least substantially similar in structure to positioning the acoustic lens 602 and the acoustic plate 606 of Fig. 6A directly next to each other. For example, the structure of the acoustic network component 702 is at least substantially similar to the structure formed by affixing the acoustic lens 602 and the acoustic plate 606 together mechanically (for example) to form an integrated structure from the two individual components. Therefore, when the acoustic lens portion 705 and the acoustic plate portion 706 are integrated together, these portions 705, 706 can be formed of different materials, such as those discussed above, that are inhomogeneously distributed within the wave-path 710. Furthermore, when integrated together, the acoustic lens portion 705 and the acoustic plate portion 706 combine at least substantially similar acoustic pulse manipulation capabilities to those of the separately positioned acoustic lens 602 and acoustic plate 606 described above for Fig. 6A. Thus, these portions 705, 706 typically possess a sound velocity component that is faster than the sound velocity component of the wave-path 710 when they are integrated together.

As discussed above, the acoustic lens portion 705 and the acoustic plate portion 706 can also be formed in a single piece of material. In this case, the resulting acoustic network component 702 is also at least substantially similar in structure to positioning the acoustic lens 602 and the acoustic plate 606 directly next to each other. However, the acoustic network component 702 is formed of one type material, such as one of those discussed above. As a result, the acoustic lens portion 705 and the acoustic plate portion 706 typically possess the same sound velocity property, which is typically faster than that of the wave-path, when the portions 705, 706 are formed in a single piece of material. Additionally, when the acoustic lens portion 705 and the acoustic plate portion 706 are formed in a single piece of material, the resulting acoustic network component 702 also combines at least substantially similar acoustic pulse manipulation capabilities to those of the separately positioned acoustic lens 702 and acoustic plate 706 described above.

Forming the acoustic network component 702 by integrating together the acoustic lens portion 705 and the acoustic plate portion 706 or forming the portions 705, 706 in the same piece of material can offer similar benefits to those discussed above with respect to the exemplary embodiment 500 of Figs. 5A-5B. For example, the single material formation of the acoustic network component 702 can provide a simple and less expensive manufacturing process in contrast to the process of manufacturing separate components 602, 606. Also, the integrated formation of the acoustic network component 702 might be implemented in smaller physical structures than the separate components 702, 706. However, as discussed above, implementing the separate components 602, 606 can offer superceding benefits in certain applications and/or under certain considerations and requirements.

In regard to the operation of the exemplary embodiments described above with respect to Figs. 3A-7B, the amount of delay or advance, respectively, that occurs to the travel of one acoustic converging pulse component with respect to other pulse components that pass through the acoustic network may depend on several factors. For example, the sound velocity component of the acoustic plate or the acoustic plate portion, respectively, in contrast to the sound velocity component of the wave-path is a factor that can affect the amount of delay or advance that occurs to the travel of the pulse component. Other factors affecting the delay or advance will be apparent to those skilled in the art based on the above discussion of the exemplary embodiments.

The acoustic network components described above with regard to Figs. 3A-7B can be constructed using various methods, which may be known in the art. For example, these components may be constructed using a casting process As another example, the components may be constructed using an injection molding process. It will be apparent to those skilled in the art, based on the above discussion of the exemplary embodiments, that other methods for constructing the acoustic network components are available, and all such methods are within the scope of the present invention.

Similarly, the wave-paths described above with regard to Figs. 3A-7B can also be constructed by various methods, which may be known in the art, depending on their structure. For example, the wave-paths may consist of an open space, a closed or partially closed space containing a medium material, or any of various forms of acoustic waveguides. It is further noted that although the acoustic network components and the wave-paths described above for Figs. 3A-7B are depicted with certain shapes (e.g., circular or conical), the shapes of these components are not limited to such, as will be apparent to those skilled in the art based on the above discussion of the exemplary embodiments.

Various modifications and additional embodiments of the present invention will become apparent to those skilled in the art based on the above discussion of the exemplary embodiments. It is to be understood that the invention is not limited to the specific exemplary embodiments disclosed and that modifications and additional embodiments may be applied to the present invention without departing from its spirit and scope as set forth in the appended claims and equivalence thereof. All such modifications and additional embodiments are intended to be included within the scope of the appended claims.

## Claims

1. An apparatus for manipulating acoustic pulses, comprising:
an acoustic wave-path (410, 510, 610, 710) having at least a first acoustic impedance, including a first sound velocity component, and structured to allow the travel of acoustic pulses; and
an acoustic network (302, 502, 702) suspended within the acoustic wave-path, comprising:
at least one acoustic lens (402, 505, 602, 705) having at least a second acoustic impedance, including a second sound velocity component, and structured to convert an acoustic plane pulse (420-1, 520-1, 620-1, 720-1) into an acoustic converging pulse (420-2, 620-2) when at least part of the acoustic plane pulse travels through the at least one acoustic lens; and
at least one acoustic plate (406, 506, 606, 706) having at least a third acoustic impedance, including a third sound velocity component, and structured to convert an acoustic converging pulse into a split acoustic converging pulse (420-3, 520-2, 620-3, 720-2) having at least two components when at least part of the acoustic converging pulse travels through the at least one acoustic plate.

2. The apparatus of claim 1, wherein the acoustic network comprises:
an acoustic lens (402, 505) having at least one convex shaped surface (403, 503), wherein the second sound velocity component of the acoustic lens is slower than the first sound velocity component of the acoustic wave-path (410, 510); and
an acoustic plate (406, 506) having at least one convex shaped surface (407) and at least one concave shaped surface (408, 508), wherein the third sound velocity component of the acoustic plate is slower than the first sound velocity component of the acoustic wave-path.

3. The apparatus of claim 2, wherein the acoustic plate (406) extends across less than an entire cross section of the acoustic wave-path (410) so that when an acoustic converging pulse (420-2) travels through the acoustic plate, the acoustic plate converts the acoustic converging pulse into a split acoustic converging pulse (420-3) having at least two components, wherein the travel of at least one of the at least two components that travels through the acoustic plate is delayed with respect to the travel of the other of the at least two components that did not travel through the acoustic plate.

4. The apparatus of at least one of the preceding claims, wherein the acoustic lens (505) and the acoustic plate (506) are integrated together so that the acoustic network (502) has at least one convex surface (503) and at least one concave surface (508).

5. The apparatus of at least one of the preceding claims, wherein the acoustic lens (505) and the acoustic plate (506) are comprised of a single piece of a material so that the acoustic network (502) has at least one convex surface (503) and at least one non-uniformly stepped surface (508).

6. The apparatus of claim 5, wherein the at least one non-uniformly stepped surface (508) is at least substantially concave shaped.

7. The apparatus of claim 1, wherein the acoustic network (702) comprises:
an acoustic lens (602, 705) having at least one concave shaped surface (603, 604, 703), wherein the second sound velocity component of the acoustic lens is faster than the first sound velocity component of the acoustic wave-path (610, 710); and
an acoustic plate (606, 706) having at least one convex shaped surface (607) and at least one concave shaped surface (608, 708), wherein the third sound velocity component of the acoustic plate is faster than the first sound velocity component of the acoustic wave-path.

8. The apparatus of claim 7, wherein the acoustic plate (606) extends across less than an entire cross section of the acoustic wave-path (610) so that when an acoustic converging pulse (620-2) travels through the acoustic plate, the acoustic plate converts the acoustic converging pulse into a split acoustic converging pulse (620-3) having at least two components, wherein the travel of at least one of the at least two components that travels through the acoustic plate is advanced with respect to the travel of the other of the at least two components that did not travel through the acoustic plate.

9. The apparatus of at least one of claims 1 through 3, 7 and 8, wherein the acoustic lens and the acoustic plate are integrated together so that the acoustic network (702) has at least two concave surfaces (703, 708).

10. The apparatus of at least one of claims 1 through 3 and 7 through 9, wherein the acoustic lens (705) and the acoustic plate (706) are comprised of a single piece of a material so that the acoustic network (702) has one concave surface (703) and at least one non-uniformly stepped surface (708).

11. The apparatus of claim 10, wherein the at least one non-uniformly stepped surface (708) is at least substantially concave shaped.

12. The apparatus of at least one of the preceding claims, wherein the acoustic wave-path (410, 510, 610, 710) is a volume of a liquid, gas, or solid.

13. The apparatus of at least one of the preceding claims, wherein the acoustic wave-path (410, 510, 610, 710) is a waveguide structured to guide the travel of acoustic pulses.

14. The apparatus of at least one of the preceding claims, wherein the at least one acoustic lens (505, 705) and the at least one acoustic plate (506, 706) are integrated together.

15. The apparatus of at least one of the preceding claims, wherein the at least one acoustic lens (505, 705) is comprised of at least a first material and the at least one acoustic plate (506, 706) is comprised of at least a second material, so that the acoustic network (502, 702) is comprised of at least two materials that are inhomogeneously distributed within the acoustic wave-path (510, 710).

16. The apparatus of at least one of the preceding claims, wherein the at least one acoustic lens (505, 705) and the at least one acoustic plate (506, 706) are comprised of a single piece of a material.

17. The apparatus of at least one of the preceding claims, wherein the acoustic lens (402, 505, 602, 705) is comprised of a material that is a polymer, silicon, a synthetic rubber, polystyrene, polyurethane, or a liquid.

18. The apparatus of at least one of the preceding claims, wherein the acoustic plate (406, 506, 606, 706) is comprised of a material that is a polymer, silicon, a synthetic rubber, polystyrene, polyurethane, or a liquid.

19. A method for manipulating acoustic pulses, comprising:
providing an acoustic wave-path (410, 510, 610, 710) having at least a first acoustic impedance, including a first sound velocity component, and structured to allow the travel of acoustic pulses;
providing at least one acoustic lens (402, 505, 602, 705), suspended within the acoustic wave-path, having at least a second acoustic impedance, including a second sound velocity component, and structured to convert an acoustic plane pulse (420-1, 520-1, 620-1, 720-1) into an acoustic converging pulse (420-2, 620-2) when at least part of the acoustic plane pulse travels through the at least one acoustic lens;
providing at least one acoustic plate (406, 506, 606, 706), suspended within the acoustic wave-path, having at least a third acoustic impedance, including a third sound velocity component, and structured to convert an acoustic converging pulse into a split acoustic converging pulse (420-3, 520-2, 620-3, 720-2) having at least two components when at least part of the acoustic converging pulse travels through the at least one acoustic plate;
transmitting an acoustic plane pulse along the acoustic wave-path;
converting the acoustic plane pulse into an acoustic converging pulse by transmitting at least part of the acoustic plane pulse through the at least one acoustic lens; and
converting the acoustic converging pulse into a split acoustic converging pulse by transmitting at least part of the acoustic converging pulse through the at least one acoustic plate.

20. The method of claim 19, wherein:
providing at least one acoustic lens comprises providing an acoustic lens (402, 505) having at least one convex shaped surface (403, 503) and a second sound velocity component that is slower than the first sound velocity component of the acoustic wave-path (410, 510);
providing at least one acoustic plate comprises providing an acoustic plate (406, 506), extending across less than an entire cross section of the acoustic wave-path, having at least one convex shaped surface (407), at least one concave shaped surface, and a third sound velocity component that is slower than the first sound velocity component of the acoustic wave-path; and
converting the acoustic converging pulse comprises converting the acoustic converging pulse (420-2) into a split acoustic converging pulse (420-3, 520-2) having at least two components, wherein the travel of at least one of the at least two components that is transmitted through the acoustic plate is delayed with respect to the travel of the other of the at least two components that is not transmitted through the acoustic plate.

21. The method of claim 19, wherein:
providing at least one acoustic lens comprises providing an acoustic lens (602, 705) having at least one concave shaped surface (603, 703) and a second sound velocity component that is faster than the first sound velocity component of the acoustic wave-path (610, 710);
providing at least one acoustic plate comprises providing an acoustic plate (606, 706), extending across less than an entire cross section of the acoustic wave-path, having at least one convex shaped surface (607), at least one concave shaped surface (608, 708), and a third sound velocity component that is faster than the first sound velocity component of the acoustic wave-path; and
converting the acoustic converging pulse (620-2) comprises converting the acoustic converging pulse into a split acoustic converging pulse (620-3, 720-2) having at least two components, wherein the travel of at least one of the at least two components that is transmitted through the acoustic plate is advanced with respect to the travel of the other of the at least two components that is not transmitted through the acoustic plate.

22. The method of one of claims 19, 20 and 21, further comprising integrating together the at least one acoustic lens (505, 705) and the at least one acoustic plate (506, 706).

23. The method of one of claims 19, 20, 21 and 22, further comprising forming the at least one acoustic lens (505, 705) and the at least one acoustic plate (506, 706) out of a single piece of a material.
